(19) Europäisches Patentamt
European Patent Office
Office européen des brevets

(11) Veröffentlichungsnummer: **0 058 954**
**A2**

(12) # EUROPÄISCHE PATENTANMELDUNG

(21) Anmeldenummer: **82101261.4**

(51) Int. Cl.³: **C 12 N 15/00**, C 07 H 21/04

(22) Anmeldetag: **19.02.82**

(30) Priorität: **25.02.81 DE 3106982**

(43) Veröffentlichungstag der Anmeldung: **01.09.82 Patentblatt 82/35**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Seliger, Hartmut, Prof.Dr.Ing., Öschwende 55, D-7900 Ulm-Lehr (DE)**
Erfinder: **Rastl, Eva, Dr., Breitenfurter Strasse 1/II/17, A-1120 Wien 12 (AT)**
Erfinder: **Swetly, Peter, Dr., Hietzinger Hauptstrasse 40 B, A-1130 Wien (AT)**

(54) **Neues Tridecadeoxynukleotid, Verfahren zu seiner Herstellung und Verwendung.**

(57) Neues Tridecadeoxynukleotid der Formel

dCCTTCTGGAACTG

und dessen Verwendung als Primer für die Herstellung von komplementären Deoxyribonukleinsäure an Leukozyten-, Fibroblasten-, Lymphoblasten- oder Immuninterferon- mRNA-Matrizen.

Das neue Nukleotid kann nach für analoge Verbindungen üblichen Verfahren hergestellt werden.

EP 0 058 954 A2

Dr. Karl Thomae GmbH
Case 5/827 - Dr.Fl./Kp.

## Neues Tridecadeoxynukleotid, Verfahren zu seiner Herstellung und Verwendung

Hochgereinigtes Interferon steht nur in äußerst geringen Mengen für medizinische Untersuchungen zur Verfügung. Um die Produktion größerer Interferonmengen zu ermöglichen und damit diesen Bedarf abzudecken, wird mit Hilfe gentechnologischer Methoden menschliches Interferon in Bakterien synthetisiert (siehe S. Nagata, H. Taira, A. Hall, L. Johnsrud, M. Streuli, J. Escödi, W. Boll, K. Cantell und C. Weissmann in Nature 284, 316 (1980)).

Dazu ist es notwendig, das Gen, das die Proteinsequenz des zu produzierenden menschlichen Leukozyteninterferons codiert, in einer geeigneten Weise in ein Bakterium zu transferieren, so daß die Genexpression und damit die Synthese des Human-Interferons im Bakterium ermöglicht wird. Dabei geht man so vor, daß man die entsprechende Deoxyribonucleinsäuresequenz in einen selbstreplizierenden Vektor (vorzugsweise ein Bakterienplasmid oder ein Bakterienvirus) integriert und dieses so modifizierte genetische Material in die Bakterienzelle einschleust. Damit die Expression dieses artfremden Gens stattfinden kann, ist es notwendig, die Deoxyribonucleinsäuresequenz in der richtigen Orientierung und im richtigen Leserahmen in den Vektor einzubauen.

Die Auswahl der für diese Gen-Manipulation geeigneten Nucleinsäure erfolgt zweckmäßigerweise auf der messenger-Ribonucleinsäure-Ebene (mRNA-Ebene), d.h. man geht zur Gewinnung der benötigten DNA so vor, daß man mit Hilfe eines Enzyms, der "Reversen Transskriptase", die zur mRNA komplementäre DNA synthetisiert. Um diese enzymatische Synthese zu initiieren, wird ein sogn. "Primer" benötigt, ein Stück DNA, dessen Basensequenz komplementär zu einem Teilbereich der betreffenden mRNA ist und das nach Zugabe zur mRNA an diesen komplementären Teilbereich in Form eines Hybrid-Doppelstranges gebunden ist.

Für den Fall, daß die betreffende mRNA poly-A-haltige Teilbereiche besitzt, wäre als Primer oligo-dT geeignet. Da jedoch die "richtige" mRNA nicht in reiner Form, sondern im Gemisch mit anderen mRNA-Sequenzen vorliegt, würde so die Transskription aller poly-A-haltigen mRNA-Sequenzen initiiert, was zu einem komplexen Gemisch von DNA-Teilstücken führen würde, das für den oben genannten Zweck nicht geeignet wäre.
Verwendet man hingegen als Primer ein Desoxy-oligonucleotid-Teilstück, das zu einer spezifischen Basensequenz der "richtigen" mRNA komplementär ist, so erhöht sich die Wahrscheinlichkeit, daß auch in Gegenwart verschiedener anderer mRNA nur diese eine gewünschte Transskription stattfindet und somit in der Reverse-Transskriptase-Reaktion die gewünschte komplementäre DNA in weitgehend einheitlicher Form synthetisiert wird.

Der folgenden Anmeldung liegt die Erkenntnis zugrunde (siehe T. Taniguchi, N. Mantei, M. Schwarzstein, S. Nagata, M. Muramatsu und C. Weissmann in Nature 285, 547 (1980)), daß allen bisher bekannten Interferon-Genen eine aus dreizehn Nucleotiden bestehende Sequenz gemeinsam ist, welche die Aminosäuren Nr. 47-50 codiert.

Eine Sequenz von dreizehn Deoxynucleotiden bindet stabil in Form eines Hybrid-Doppelstranges (siehe M. Mevarech, B.E. Noyes und K. L. Agarwal in J. Biol. Chem. 254, 7472 (1979)) an alle jene mRNA-Moleküle, welche eine dazu komplementäre Sequenz enthalten, jedoch nicht an die im vorliegenden Gemisch überwiegend enthaltenen mRNA-Moleküle, die diese spezifische Sequenz nicht enthalten. Verwendet man deshalb in der Reverse-Transskriptase-Reaktion eine derartig spezifische DNA-Sequenz als Primer, so erfolgt dadurch im Gemisch verschiedener mRNA eine Auslese der interferon-spezifischen mRNA-Spezies, und die Wahrscheinlichkeit, auf diese Weise interferon-spezifische komplementäre DNA zu erhalten, die frei von nicht-interferon-spezifischer DNA ist, wird wesentlich erhöht.

Gegenstand der vorliegenden Erfindung ist somit das neue Tridecadeoxynukleotid der Formel

dCCTTCTGGAACTG

ein Verfahren zu seiner Herstellung und seine Verwendung, insbesondere als "Primer" bei der enzymatischen Synthese von Deoxyribonukleinsäure (DNA) an messenger-Ribonukleinsäure (mRNA)-Matrizen.

Erfindungsgemäß erhält man das neue Tridecadeoxynukleotid durch Verknüpfung geeigneter partiell geschützter Oligonukleotidblöcke, insbesondere von zwei geeigneten Oligonukleotidfragmenten, z.B. durch Verknüpfung eines entsprechenden Tetrameren mit einem entsprechenden Nonameren, unter Verwendung eines Kondensationsmittels.

Als Kondensationsmittel kommt ein die Säure aktivierendes oder wasserentziehendes Mittel wie Sulfonyltetrazolide, Sulfonyltriazolide, Sulfonylimidazolide, Sulfonylchloride oder Carbodiimide, z. Mesitylen-sulfonyltetrazolid oder N,N'-Dicyclohexylcarbodiimid in Betracht.

Als Schutzgruppen kommen beispielsweise für die 5'-terminale OH-Gruppe vorzugsweise eine säurelabile Schutzgruppe wie die Trityl-, Monomethoxytrityl-, Dimethoxytrityl-, 1-Ethoxyethyl- oder 4-Methoxy-tetrahydro-pyran-4-yl-gruppe, für den Phosphatrest die Trichlorethyl-, Cyanethyl- oder p-Chlorphenylgruppe und
für die Aminofunktionen der Basen die Isobutyloxycarbonyl-, Benzoyl-, Anisoyl- oder Isobutyrylgruppe in Betracht.

Die Umsetzung erfolgt in einem Lösungsmittel, vorzugsweise in einem wasserfreien Lösungsmittel, z.B. in absolutem Pyridin, in Gegenwart eines Kondensationsmittels, vorzugsweise Mesitylensulfonyltetrazolid, bei Temperaturen zwischen 0 und 50°C, vorzugsweise jedoch bei Raumtemperatur.

Von dem so erhaltenen voll geschützten Tridecameren werden anschließend die Schutzreste abgespalten. So wird ein säurelabiler Schutzrest wie die Dimethoxy-tritylgruppe in einem Lösungsmittel, z.B. in Methanol/Chloroform, und in Gegenwart einer Säure wie Benzolsulfonsäure zweckmäßigerweise bei 0°C und anschließend die übrigen Schutzreste, z.B. die Benzoyl-, Isobutyryl- oder p-Chlorphenylphosphoryl-gruppen, durch Behandeln mit einer Base, z.B. Pyridin/konz. Ammoniak, bei Temperaturen zwischen 20 und 80°C, vorzugsweise jedoch zuerst bei 35°C und dann bei 55°C, abgespalten.

Die nachfolgenden Beispiele sollen die Erfindung näher erläutern:

Herstellung der Ausgangsprodukte:

Legende:

| | | |
|---|---|---|
| DMTr | = | p,p'-Dimethoxy-triphenylmethyl |
| p̣ | = | p-Chlorphenylphosporyl |
| CE | = | ß-Cyanoethyl |
| ibu | = | Isobutyryl |
| bz | = | Benzoyl (am Basenstickstoff) |
| Bz | = | Benzoyl (am Furanylsauerstoff) |
| DTT | = | Dithiothreitol |
| EDTA | = | Äthyldiamintetraacetat |
| RNA | = | Ribonukleinsäure |
| DNA | = | Deoxyribonukleinsäure |
| dNTP | = | Gemisch aus 4 Deoxynucleotid-triphosphaten des Adenosins, Thymidins, Guanosins und Cytidins |
| cDNA | = | DNA, welche komplementär zu messenger RNA ist |

**Beispiel A**

5'-Dimethoxytrityldesoxythymidin-3'-p-chlorphenyl-ß-cyanoethyl-phosphat DMTrdTp̣(CE)

1,59 g (3 mMol) 5'-Dimethoxytrityl-thymidin (hergestellt nach H. Schaller et al., J. Amer. chem. Soc. 85, 3821 (1963)) werden in etwa 20 ml absolutem Acetonitril aufgelöst. Zur Entfernung von Wasserspuren werden mehrmals 3 - 4 ml Acetonitril abgezogen und durch absolutes Acetonitril ersetzt (≙ absolutieren).

Die trockene Lösung wird mit 1 ml (ca. 12,5 mMol) N-Methyl-imidazol versetzt. 2,8 g (10 mMol) p-Chlorphenyl-ß-cyanoethyl-phosphoryl-chlorid, gelöst in 5 ml absolutem Acetonitril, werden langsam unter Kühlung zugetropft. Die Mischung wird 2 Stunden

bei 20°C reagieren lassen, wonach dünnschichtchromatographisch eine vollständige Umsetzung zum obengenannten Produkt festgestellt wird.

Zum Reaktionsabbruch wird die Mischung im Eisbad gekühlt und dann 5 ml 1 M Ammoniumhydrogencarbonatlösung langsam zugetropft. Die Mischung läßt man 1 Stunde bei Raumtemperatur stehen. Sie wird anschließend einrotiert, in 20 ml Chloroform aufgenommen und 3 mal mit etwa dem gleichen Volumen 0,1 M Ammoniumhydrogencarbonatlösung gewaschen, zum Schluß 1 mal mit dem gleichen Volumen Wasser. Die Chloroformphase wird über Natriumsulfat getrocknet, filtriert und einrotiert. Der Rückstand ist weitgehend reines 5'-Dimethoxytrityl-desoxythymidin-3'-p-chlorphenyl-ß-cyanoethylphosphat. Zur Hochreinigung wird die Verbindung mit Hilfe der präparativen Flüssigchromatographie (Prep-LC 500, Fa. Waters) auf einer Kieselgelsäule mit 2,5 % Methanol in Methylenchlorid chromatographiert.
Ausbeute: 1,8 g (ca. 80 % der Theorie).
Charakterisierung:

UV in Methanol: $\lambda_{max}$ = 268 nm; $\lambda_{min}$ = 250 nm
in Perchlorsäure/Ethanol 9 : 1 : $\lambda_{max}$ = 500 nm;
$A_{280}/A_{500}$ = 0,13

$R_f$ der DC auf Kieselgel
in Chloroform/Methanol 9 : 1 = 0,48
in Chloroform/Ethanol 1 : 1 = 0,77
in Chloroform/Methanol 9 : 1 nach sechsstündiger Behandlung mit Triethylamin $R_f$ von 0,48 nach 0,0; nach Behandlung mit 2 % Benzolsulfonsäure in Chloroform/Methanol 7/3 $R_f$ von 0,48 nach 0,26.

Beispiel B

$N^2,O^{3'},O^{5'}$-Triisobutyryl-desoxyguanosin $dG^{ibu}$

2,67 g (10 mMol) Desoxyguanosin (hergestellt analog H. G. Khorana et al., J. Mol. Biol. 72, 251 (1972)) werden in Dioxan suspendiert und lyophilisiert. Das so vorgetrocknete Desoxyguanosin wird noch 3 mal aus einer Suspension in je 10 ml absolutem Pyridin absolutiert. Anschließend wird eine Suspension in einer Mischung aus 60 ml Chloroform und 7 ml Pyridin bereitet. Zu dieser Suspension werden 8 ml (80 mMol) Isobuttersäurechlorid unter Kühlung und Rühren langsam zugetropft. Die Suspension wandelt sich dabei langsam in eine klare Lösung um. Nach 2 Stunden Reaktionszeit wird die Lösung mit 20 ml Wasser oder wahlweise 0,1 M Ammoniumhydrogencarbonatlösung unter Kühlung versetzt. Die organische Phase wird einrotiert, bis ein zäher, gummiartiger Rückstand verbleibt, der anschließend in 100 ml Ethanol aufgenommen wird. Ein Dünnschichtchromatogramm auf Kieselgel in Ethanol/Chloroform = 1/1 zeigt vollständigen Umsatz des Desoxyguanosins zu $N,O^{3'},O^{5'}$-Triisobutyryl-desoxyguanosin an.

Die ethanolische Lösung des Zwischenprodukts wird mit 100 ml 2 N Natronlauge bei $0^oC$ 15 Minuten behandelt, dann schnell mit Dowex 50 - Ionenaustauscher (Pyridiniumform) neutralisiert. Der Ionenaustauscher wird abfiltriert und gründlich mit Methanol/Pyridin und einer warmen Methanol/Wasser-Mischung gewaschen. Die Waschlösungen werden mit dem Filtrat vereinigt. Die vereinigten Lösungen werden am Rotationsverdampfer bis zur Trockne eingeengt. Der Rückstand wird in heißem Wasser gelöst; er kristallisiert beim Abkühlen.
Ausbeute: 2,7 g (80 % der Theorie).
Charakterisierung:
UV in Methanol: $\lambda_{max}$ = 280 nm sowie breites Maximum bei 254 - 260 nm; $\lambda_{min}$ = 272, 227 nm
$A_{260}/A_{280}$ = 1,36

## Beispiel C

a) 5'-Dimethoxytrityl-$N^2$-isobutyryl-desoxyguanosin $DMTrdG^{ibu}$

2,7 g (8 mMol) $N^2$-Isobutyryldesoxyguanosin und 2,85 g (8,5 mMol) Dimethoxytritylchlorid werden unter Zusatz einer kleinen Menge 4-Dimethylaminopyridin (DMAP) als Katalysator 2 Stunden bei Raumtemperatur in 20 ml absolutem Pyridin reagieren lassen. Der Umsatz wird durch DC auf Kieselgel in Ethanol/Chloroform 1 : 1 geprüft. Falls der Umsatz nicht vollständig ist, kann mit einem weiteren Zusatz von ca. 0,5 mMol Dimethoxytritylchlorid eine weitere Stunde reagiert werden. Anschließend werden 20 ml eisgekühltes Wasser zugesetzt. Die Wasser-Pyridin-Lösung wird 3 mal mit je 30 ml Chloroform extrahiert. Die Chloroformphase wird über Natriumsulfat getrocknet, filtriert und unter Zusatz von Toluol einrotiert, um Pyridin zu entfernen. Der Rückstand wird in wenig Chloroform aufgenommen und mittels präparativer Flüssigchromatographie an Kieselgel mit 4 % Methanol in Methylenchlorid getrennt.

Ausbeute: 4 g (80 % der Theorie).

Charakterisierung:

UV in Methanol: $\lambda_{max}$ = 277, 255 - 260 nm;

$\lambda_{min}$ = 272, 235 nm.

in Perchlorsäure/Ethanol 9 : 1 : $\lambda_{max}$ = 500 nm;

$A_{260}/A_{500}$ = 0,26

$R_f$ der DC auf Kieselgel

in Chloroform/Methanol 9 : 1 = 0,28

in Chloroform/Ethanol 1 : 2 = 0,65

b) 5'-Dimethoxytrityl-N$^2$-isobutyryl-desoxyguanosin-3'-p-chlorphenyl-ß-cyanoethyl-phosphat
DMTrdG$^{ibu}$p(CE)

Die Darstellung erfolgt analog Beispiel A ausgehend von 5'-Dimethoxytrityl-N$^2$-isobutyryl-desoxyguanosin. Die Reaktionskontrolle durch DC in Chloroform/Ethanol 1:1 zeigt vollständigen Umsatz an. Die Hochreinigung wird mit Hilfe der präparativen Flüssigchromatographie an Kieselgel in 3,5 % Methanol in Methylenchlorid durchgeführt und ergibt bei einem Ansatz von 5 mMol Vorstufenmaterial 3,6 g (81 % der Theorie) Reinprodukt.

Charakterisierung:

UV in Methanol: $\lambda_{max}$ = 277, 254 - 260 nm; $\lambda_{min}$ = 272, 235 nm in Perchlorsäure/Ethanol 9 : 1;
$\lambda_{max}$ = 500 nm;
$A_{280}/A_{500}$ = 0,27

$R_f$ der DC auf Kieselgel
in Chloroform/Methanol 9 : 1 = 0,43
in Chloroform/Ethanol 1 : 1 = 0,62

## Beispiel D

5'-Dimethoxytrityl-N$^4$-benzoyl-desoxycytidin-3'-p-chlorphenyl-ß-cyanoethyl-phosphat
DMTrdC$^{bz}$p(CE)

Die Darstellung erfolgt analog Beispiel A ausgehend von 5'-Dimethoxytrityl-N$^4$-benzoyl-desoxycytidin (hergestellt nach H. Schaller et al., J. Amer. chem. Soc. 85, 3821 (1963), die Hochreinigung erfolgt durch präparative Flüssigchromatographie an Kieselgel mit 2,5 % Methanol in Methylenchlorid). Die Reaktionskontrolle erfolgt durch DC in Chloroform/Methanol 9 : 1 und zeigt vollständigen Umsatz der Vorstufe an. Zur Hochreinigung wurde das Produkt durch präparative Flüssigchromatographie auf einer Kieselgelsäule in 2 % Methanol in Methylenchlorid chromatographiert.
Ausbeute: 85 % der Theorie.
Charakterisierung:
UV in Methanol: $\lambda_{max}$ = 305, 260 nm; $\lambda_{min}$ = 285, 240 nm
$R_f$ der DC auf Kieselgel
in Chloroform/Methanol 9 : 1 = 0,67

Beispiel E

5'-Dimethoxytrityl-$N^6$-benzoyl-desoxyadenosin-3'-p-chlorphenyl-ß-cyanoethylphosphat
DMTrd$A^{bz}$p(CE)

Die Darstellung erfolgt analog Beispiel A ausgehend von 5'-Dimethoxytrityl-$N^6$-benzoyl-desoxyadenosin (hergestellt nach H. Schaller et al., J. Amer. chem. Soc. 85, 3821 (1963)), die Hochreinigung erfolgt durch präparative Flüssigchromatographie an Kieselgel mit 3,5 % Methanol in Methylenchlorid). Die Reaktionskontrolle durch DC in Chloroform/Methanol 9 : 1 zeigt vollständigen Umsatz der Vorstufe an. Zur Hochreinigung wird das Produkt durch präparative Flüssigchromatographie auf einer Kieselgelsäule in 2,5 % Methanol in Methylenchlorid chromatographiert.
Ausbeute: 80 % der Theorie
Charakterisierung:
UV in Methanol: $\lambda_{max}$ = 280 nm; $\lambda_{min}$ = 256 nm.
$R_f$ der DC auf Kieselgel in Chlorofom/Methanol 9 : 1 = 0,63.

Beispiel F

$N^2$-Isobutyryl-$O^{3'}$-benzoyl-desoxyguanosin

Zur Benzoylierung wird 1 mMol 5'-Dimethoxytrityl-$N^2$-isobutyryl-desoxyguanosin (siehe Beispiel B) in 15 ml absolutem Pyridin gelöst. 1,2 mMol Benzoylchlorid werden bei Kühlung auf $0^oC$ langsam zugetropft. Die Reaktion ist in 4 Stunden beendet (Überprüfung durch DC an Kieselgel in Chloroform/Methanol 9 : 1). Zum Reaktionsgemisch werden dann 10 ml Wasser oder 0,1 M Ammoniumhydrogencarbonatlösung zugesetzt. Anschließend wird 3 mal mit Chloroform extrahiert. Die Chloroform-Extrakte werden mehrmals mit Wasser rückextrahiert und anschließend über Natriumsulfat getrocknet. Die Chloroformlösung wird filtriert, eingeengt und anschließend zum Entfernen von Pyridin mit Toluol am Rotationsverdampfer zur Trockne gebracht.

Zum Entfernen der Dimethoxytritylschutzgruppe wird der Rückstand anschließend in einer Mischung aus 2 % Benzolsulfonsäure in Chloroform/Methanol 7 : 3 3 Minuten bei $0^oC$ behandelt. Danach werden 5 ml 5%ige Ammoniumhydrogencarbonatlösung zugesetzt. Die Chloroformphase wird getrocknet, filtriert, eingeengt und mit Hilfe der präparativen Flüssigchromatographie auf Kieselgel in 3,5 % Methanol in Methylenchlorid gereinigt.
Ausbeute: 80 % der Theorie.
Charakterisierung:
UV in Methanol: $\lambda_{max}$ = 277, 255 - 260 nm; $\lambda_{min}$ = 272, 235 nm.
$R_f$ der DC auf Kieselgel
in Chloroform/Methanol 9 : 1 = 0,20

Beispiel G

Abspaltung der Dimethoxytritylgruppe aus Dimethoxytrityl-nucleosid-3'-p-chlorphenyl-ß-cyanoethyl-phosphat

Die Ausgangsverbindung wird unter Eiskühlung mit einer 2%igen Lösung von Benzolsulfonsäure in Chloroform/Methanol 7 : 3 versetzt und 3 - 4 Minuten gerührt. Anschließend wird durch Zugabe von 5%iger Ammoniumhydrogencarbonatlösung neutralisiert. Die organische Phase wird abgetrennt und die wäßrige 1 mal mit Chloroform extrahiert. Die vereinigte Chloroformphase wird getrocknet und einrotiert. Der Rückstand wird in wenig Chloroform gelöst und entweder über eine Kieselgelsäule ("short-column", Durchmesser: Länge = ca. 1 : 5 bis 1 : 8; Laufmittel: Chloroform + 0 - 7 % Methanol) oder durch präparative Flüssigchromatographie auf Kieselgel (Laufmittel: Chloroform/Methanol, Methanolgehalt durch HPLC-Vorversuch ermitteln) gereinigt.
Ausbeute: 80 - 90 % der Theorie.
Die detritylierten Monomeren werden als Chloroform-Stammlösung bei $-20^{o}C$ aufbewahrt.

Beispiel H

Abspaltung der ß-Cyanoethylgruppe aus Dimethoxytrityl-nucleosid-3'-p-chlorphenyl-ß-cyanoethyl-phosphat

Die für die Kondensation benötigte Menge der Ausgangsverbindung wird in wenig Pyridin gelöst, absolutiert und mit einem 50- bis 100-fachen Überschuß an Triethylamin behandelt. Nach 2 bis 6 Stunden ist die ß-Cyanoethylgruppe quantitativ abgespalten (Kontrolle durch DC auf Kieselgel in Chloroform/Methanol 9 : 1; Produkt hat $R_f$ = 0,0). Überschüssiges Triethylamin und das entstandene Acrylnitril werden im Vakuum abgezogen. Der Rückstand wird in wenig absolutem Pyridin aufgenommen.

Beispiel I

$N^2$-Isobutyryl-desoxyguanosin-3'-p-chlorphenylphosphoryl-5'-($N^2$-isobutyryl)-desoxyguanosin-3'-p-chlorphenyl-ß-cyanoethylphosphat $dG^{ibu}\underset{\bullet}{p}G^{ibu}\underset{\bullet}{p}(CE)$

0,6 mMol 5'-Dimethoxytrityl-$N^2$-isobutyryl-desoxyguanosin-3'-p-chlorphenylphosphat werden in 10 ml absolutem Pyridin gelöst und mehrmals absolutiert. Eine ebenso vorbehandelte Pyridinlösung von 0,4 mMol $N^2$-Isobutyryl-desoxyguanosin-3'-p-chlorphenyl-ß-cyano-ethylphosphat wird zugegeben und die Mischung auf ein Volumen von ca. 10 ml eingeengt. Anschließend werden 1,5 mMol Mesitylensulfonyltetrazolid zugesetzt und nochmals etwas Pyridin abgezogen. Die Umsatzkontrolle erfolgt durch DC auf Kieselgel in Chloroform/Methanol 9 : 1, erstmals nach 30 Minuten Reaktionszeit bei Raumtemperatur. Nach 45 Minuten zeigt das Dünnschichtchromatogramm Verschwinden der Hydroxylkomponente $dG^{ibu}\underset{\bullet}{p}(CE)$ an. Die Kondensation wird alsbald unter Eiskühlung mit 10 ml 5%iger Ammoniumhydrogencarbonatlösung abgebrochen und das Reaktionsgemisch 1 Stunde bei Raumtemperatur stehengelassen.

Zur Aufarbeitung wird das Gemisch 2 mal mit je 10 ml Chloroform extrahiert. Die vereinigten Chloroform-Phasen werden 1 mal mit 0,1 M Ammoniumhydrogencarbonatlösung und 1 mal mit 10 ml Wasser gewaschen. Anschließend wird die Chloroformlösung mit Natriumsulfat getrocknet und einrotiert. Durch mehrmaliges Einrotieren mit Toluol und Methanol wird restliches Pyridin aus der Lösung entfernt. Der Rückstand ist rohes 5'-Dimethoxytrityl-$N^2$-isobutyryl-desoxyguanosin-3'-p-chlorphenylphosphoryl-5'-($N^2$-isobutyryl)-desoxyguanosin-3'-p-chlorphenyl-ß-cyanoethyl-phosphat.

Soll die Reinigung auf der Stufe dieses Produkts erfolgen, dann chromatographiert man den in wenig Chloroform aufgenommenen Rückstand entweder auf einer Kieselgel-"short column" (z.B. Kieselgel 60 Merck Nr. 7733 bzw. 7734 und äquivalente Kieselgel-Sorten anderer Firmen) in Chloroform mit Gradient 0 - 7 % Methanol

oder auf einem präparativen LC-System mit Kieselgelsäule mit einem Chloroform-Methanol-Gemisch als Eluens, dessen Zusammensetzung durch analyt. HPLC vorher festgelegt wird.

Die tritylierten Oligomeren charakterisiert man durch ihr UV-Spektrum in Methanol ($\lambda_{max}$ und $\lambda_{min}$ wie das detritylierte Derivat, siehe unten) und in Perchlorsäure/Ethanol 9 : 1 ($\lambda_{max}$ = 500 nm; Bestimmung von $A_{280}/A_{500}$) sowie durch ihre $R_f$-Werte der DC auf Kieselgel in Chloroform/Methanol 9 : 1.

Abspaltung der Dimethoxytrityl-Schutzgruppe:

Die Abspaltung der Dimethoxytrityl-Schutzgruppe kann ohne Reinigung des vorher genannten Zwischenprodukts in folgender Weise durchgeführt werden: Der Rückstand der Aufarbeitung wird 3 Minuten bei 0°C mit einer 2%igen Lösung von Benzolsulfonsäure in Chloroform/Methanol 7 : 3 behandelt und danach schnell mit 10 ml 5%iger Ammoniumhydrogencarbonatlösung neutralisiert. Die Phasen werden getrennt und die wäßrige nochmals mit 10 ml Chloroform extrahiert. Die vereinigten Chloroform-Phasen werden über Natriumsulfat getrocknet, filtriert und eingeengt. Das Konzentrat wird auf eine Kieselgelsäule gegeben (2 x 7 cm, Kieselgel 0,2 - 0,5 mm von Macherey-Nagel oder Merck Nr. 7733). Eluiert wird mit Chloroform + 0 - 9 % Methanol (stufenweise Erhöhung der Methanolkonzentration; Durchflußgeschwindigkeit 80 - 100 ml/ Stunde; Fraktionen je 15 ml; Kontrolle der Trennung durch Dünnschichtchromatographie an Kieselgel in Chloroform/Methanol 9 : 1). Die Produktfraktionen werden vereinigt, einrotiert, in einem abgemessenen Volumen Chloroform gelöst und bei -20°C aufbewahrt.
Ausbeute: 62 % der Theorie.
Charakterisierung:
UV in Methanol: $\lambda_{max}$ = 277, 262 - 255 nm; $\lambda_{min}$ = 272, 235 nm
$A_{260}/A_{280}$ = 1,38
$R_f$-Werte der DC auf Kieselgel
in Chloroform/Methanol 9 : 1 = 0,15
in Chloroform/Methanol 8 : 2 = 0,52

Abspaltung der ß-Cyanoethyl-Schutzgruppe:

Falls Oligomere des Typs 5'-Dimethoxytrityl-nucleosid$^1$-3'-p-chlorphenylphosphoryl-5'-nucleosid$^2$-3'-p-chlorphenyl-phosphat benötigt werden, geht man von den gereinigten tritylierten Oligomeren aus und spaltet die ß-Cyanoethyl-Schutzgruppe analog Beispiel H ab. Das Produkt kann ohne weitere Reinigung verwendet werden; die Ausbeute der Decyanoethylierung ist nahezu quantitativ. Die Charakterisierung erfolgt wie oben für die tritylierten Dimeren beschrieben.

Analog wurden folgende Verbindungen hergestellt:

5'-Dimethoxytrityl-N$^4$-benzoyl-desoxycytidin-3'-p-chlorphenyl-phosphoryl-5'-(N$^4$-benzoyl-) desoxycytidin-3'-p-chlorphenyl-phosphat (Abkürzung: DMTrdC$^{bz}$pC$^{bz}$p$^-$)

Desoxythymidin-3'-p-chlorphenylphosphoryl-5'-desoxythymidin-3'-p-chlorphenyl-ß-cyanoethyl-phosphat (Abkürzung: dTpTp(CE))

5'-Dimethoxytrityl-N$^4$-benzoyl-desoxycytidin-3'-p-chlorphenyl-phosphoryl-5'-desoxythymidin-3'-p-chlorphenylphosphat (Abkürzung: DMTrdC$^{bz}$pTp$^-$)

5'-Dimethoxytrityl-N$^6$-benzoyl-desoxyadenosin-3'-p-chlorphenyl-phosphoryl-5'- (N$^6$-benzoyl-) desoxyadenosin-3'-p-chlorphenyl-phosphat
(Abkürzung: DMTrdA$^{bz}$pA$^{bz}$p$^-$)

Desoxythymidin-3'-p-chlorphenylphosphoryl-5'- (N$^2$-isobutyryl-, O$^{3'}$-benzoyl-) desoxyguanosin (Abkürzung: dTpG$_{Bz}^{ibu}$)

## Beispiel K

$DMTrdC^{bz}pTpG^{ibu}_{Bz}$

0,27 mMol $DMTrdC^{bz}p^{-}$ werden in 10 ml absolutem Pyridin gelöst und mehrmals mit Pyridin absolutiert. Dann werden 0,18 mMol $dTpG^{ibu}_{Bz}$ zugesetzt und nochmals absolutiert. Anschließend werden 0,7 mMol Mesitylensulfonyltetrazolid eingeführt und nochmals etwas Pyridin abgezogen. Die Reaktion wird 2 Stunden bei Raumtemperatur durchgeführt.
Der Reaktionsabbruch und die Aufarbeitung erfolgt analog Beispiel I.
Das Rohprodukt wird in wenig Chloroform aufgenommen und säulenchromatographisch getrennt (Säule: 2 x 10 cm; Kieselgel 60 Fa. Merck Nr. 7733 Laufmittel: Chloroform + 0 - 5 % Methanol).
Ausbeute: 65 % der Theorie.
Die Abspaltung der Dimethoxytrityl-Schutzgruppe erfolgt analog Beispiel I.
Ausbeute: 80 % der Theorie.
Charakterisierung:
UV in Methanol: $\lambda_{max}$ = 258 nm; $\lambda_{min}$ = 247 nm

## Beispiel L

$DMTrdC^{bz}pTpG^{ibu}pG^{ibu}p^{-}$

0,2 mMol $DMTrdC^{bz}pTp^{-}$ (dargestellt analog Beispiel I) werden in 10 ml absolutem Pyridin gelöst und mehrmals mit Pyridin absolutiert. Dann werden 0,092 Mol $dG^{ibu}pG^{ibu}p(CE)$ in Pyridin unter nochmaligem Absolutieren zugesetzt, anschließend 0,4 mMol Mesitylensulfonyltetrazolid. Die Reaktion erfolgt 1,5 Stunden bei Raumtemperatur.
Der Reaktionsabbruch und die Aufarbeitung erfolgt analog Beispiel I.

Das Rohprodukt wird in wenig Chloroform aufgenommen und säulenchromatographisch getrennt (Säule: 2 x 10 cm; Kieselgel 60 Fa. Merck Nr. 7733; Laufmittel: Chloroform + 0 - 7 % Methanol). Decyanoethylierung wie auf Seite 12 beschrieben. Ausbeute: 75 % der Theorie.
Charakterisierung:
UV in Methanol: $\lambda_{max}$ = 260 nm; $\lambda_{min}$ = 244 nm.

Analog wurden folgende Verbindungen hergestellt:

$DMTrdC^{bz}pC^{bz}pTpTp^{-}$

$DMTrdC^{bz}pTpG^{ibu}pG^{ibu}p^{-}$

## Beispiel M

$dA^{bz}pA^{bz}pC^{bz}pTpG^{ibu}_{Bz}$

0,75 mMol $DMTrdA^{bz}pA^{bz}p^{-}$ werden in 10 ml absolutem Pyridin gelöst und mehrmals mit Pyridin absolutiert. Dann werden 0,177 mMol $dC^{bz}pTpG^{ibu}_{Bz}$ in Pyridin unter nochmaligem Absolutieren zugesetzt, anschließend 0,55 mMol Mesitylensulfonyltetrazolid. Die Reaktion erfolgt 2,5 Stunden bei Raumtemperatur.

Der Reaktionsabbruch und die Aufarbeitung erfolgt analog Beispiel I. Das Rohprodukt wird in wenig Chloroform aufgenommen und säulenchromatographisch getrennt (Säule: 2 x 10 cm; Kieselgel 60 Fa. Merck Nr. 7733; Laufmittel: Chloroform + 0 - 5 % Methanol). Ausbeute: 80 % der Theorie.
Charakterisierung:
UV in Methanol: $\lambda_{max}$ = 295 (Schulter), 263 nm; $\lambda_{min}$ = 255 nm.

Die Detritylierung erfolgt analog Beispiel G. Das Produkt wird ohne nochmalige Säulentrennung für weitere Versuche eingesetzt.

Beispiel N

$dC^{bz}\underset{\bullet}{p}T\underset{\bullet}{p}G^{ibu}\underset{\bullet}{p}G^{ibu}\underset{\bullet}{p}A^{bz}\underset{\bullet}{p}A^{bz}\underset{\bullet}{p}C^{bz}\underset{\bullet}{p}T\underset{\bullet}{p}G^{ibu}_{Bz}$

0,024 mMol $DMTrdC^{bz}\underset{\bullet}{p}T\underset{\bullet}{p}G^{ibu}\underset{\bullet}{p}G^{ibu}\underset{\bullet}{p}^{-}$ werden in 5 ml absolutem Pyridin gelöst und mehrmals mit Pyridin absolutiert. Dann werden 0,016 mMol $dA^{bz}\underset{\bullet}{p}A^{bz}\underset{\bullet}{p}C^{bz}\underset{\bullet}{p}T\underset{\bullet}{p}G^{ibu}_{Bz}$ in Pyridin unter nochmaligem Absolutieren zugesetzt, anschließend 0,2 mmMol Mesitylensulfonyltetrazolid. Die Reaktion erfolgt 2 Stunden bei Raumtemperatur.

Der Reaktionsabbruch und die Aufarbeitung erfolgt analog Beispiel I.
Aus dem Rohprodukt wird die Dimethoxytritylgruppe analog Beispiel G abgespalten.
Das hierbei gewonnene Rohmaterial wird in wenig Chloroform aufgenommen und auf eine DC-Aluminiumfolie (20 x 20 cm, Merck Kieselgel 60 / $F_{254}$) aufgetragen und das Chromatogramm in Chloroform/Methanol 9 : 1 entwickelt. Die bei $R_f$ = ca. 0,2 liegende Produktzone wird ausgeschnitten und in einer "line elution"-Ap Apparatur (Fa. Desaga) mit Chloroform/Methanol 1 : 1 eluiert.
Das Eluat wird zur Trockne eingedampft.
Ausbeute: 34 % der Theorie.
Charakterisierung:
UV in Methanol: $\lambda_{max}$ = 258 nm; $\lambda_{min}$ = 240 nm.

Beispiel O

$dG^{ibu}\underset{\bullet}{p}A^{bz}\underset{\bullet}{p}(CE)$

Hergestellt analog Beispiel I durch Umsetzung von 1,5 mMol $DMTrdG^{ibu}\underset{\bullet}{p}^{-}$ und 1 mMol $dA^{bz}\underset{\bullet}{p}(CE)$ mit 3,8 mMol Mesitylensulfonyltetrazolid.

Ausbeute: 36 % der Theorie.
Charakterisierung:
UV in Methanol: $\lambda_{max}$ = 276 nm; $\lambda_{min}$ = 267 nm.
$R_f$ der DC auf Kieselgel in Chloroform/Methanol 9 : 1 = 0,21 und 0,30 (Diastereomere)

## Beispiel P

DMTrdA$^{bz}$p̣C$^{bz}$p̣(CE)

Hergestellt analog Beispiel I durch Umsetzung von 0,8 mMol DMTrdA$^{bz}$p$^{-}$ und 0,6 mMol dC$^{bz}$p̣(CE) mit 2,4 mMol Mesitylensulfonyltetrazolid.
Ausbeute: 73 % der Theorie.
Charakterisierung:
UV in Methanol: $\lambda_{max}$ = 263 nm, $\lambda_{min}$ = 248 nm
$R_f$ der DC auf Kieselgel in Chloroform/Methanol 9 : 1 = 0,57

## Beispiel Q

DMTrdG$^{ibu}$p̣G$^{ibu}$p̣A$^{bz}$p̣$^{-}$

Hergestellt analog Beispiel I durch Umsetzung von 0,2 mMol DMTrdG$^{ibu}$p̣$^{-}$ und 0,144 mMol dG$^{ibu}$p̣A$^{bz}$p̣(CE) mit 0,6 mMol Mesitylensulfonyltetrazolid.
Ausbeute: 60 % der Theorie.
Charakterisierung:
UV in Methanol: $\lambda_{max}$ = 278 nm, $\lambda_{min}$ = 268 nm
$R_f$ der DC auf Kieselgel in Chloroform/Methanol 9 : 1 = 0,21

Beispiel R

dTpTpC$^{bz}$pTp(CE)

Hergestellt analog Beispiel L durch Umsetzung von 0,1 mMol DMTrdTpTp$^{-}$ und 0,07 mMol dC$^{bz}$pTp(CE) mit 0,3 mMol Mesitylensulfonyltetrazolid.
Ausbeute: 30 % der Theorie.
Charakterisierung:
UV in Methanol: $\lambda_{max}$ = 262 nm, $\lambda_{min}$ = 243 nm
$R_f$ der DC auf Kieselgel
in Methanol/Chloroform 9 : 1 = 0,30

Beispiel S

dA$^{bz}$pC$^{bz}$pTpG$^{ibu}_{Bz}$

Hergestellt analog Beispiel L durch Umsetzung von 0,2 mMol DMTrdA$^{bz}$pC$^{bz}$p$^{-}$ und 0,14 mMol dTpG$^{ibu}_{Bz}$ mit 0,6 mMol Mesitylensulfonyltetrazolid.
Ausbeute: 61 % der Theorie.
Charakterisierung:
UV in Methanol: $\lambda_{max}$ = 262 nm; $\lambda_{min}$ = 247 nm
$R_f$ der DC auf Kieselgel
in Chloroform/Methanol 9 : 1 = 0,46

Beispiel T

DMTrdC$^{bz}$pC$^{bz}$pTpTpC$^{bz}$pTp$^{-}$

Hergestellt analog Beispiel N durch Umsetzung von 0,035 mMol DMTrdC$^{bz}$pC$^{bz}$p$^{-}$ mit 0,021 mMol dTpTpC$^{bz}$pTp(CE) und 0,2 mMol Mesitylensulfonyltetrazolid.

Ausbeute: 75 % der Theorie.
Charakterisierung:
UV in Methanol: $\lambda_{max}$ = 262 nm; $\lambda_{min}$ = 247 nm
$R_f$ der DC auf Kieselgel
in Chloroform/Methanol 9 : 1 = 0,48

Beispiel U

$dG^{ibu}pG^{ibu}pA^{bz}pA^{bz}pC^{bz}pTpG^{ibu}_{Bz}$

Hergestellt analog Beispiel N durch Umsetzung von 0,036 mMol $DMTrdG^{ibu}pG^{ibu}pA^{bz}p^{-}$ mit 0,022 mMol $dA^{bz}pC^{bz}pTpG^{ibu}_{Bz}$ und 0,2 mMol Mesitylensulfonyltetrazolid.
Ausbeute: 25 % der Theorie.
Charakterisierung:
UV in Methanol: $\lambda_{max}$ = 278 nm; $\lambda_{min}$ = 268 nm.
$R_f$ der DC auf Kieselgel
in Chloroform/Methanol 9 : 1 = 0,24

Beispiel 1

d C C T T C T G G A A C T G

6 µMol $DMTrdC^{bz}pC^{bz}pTpTp^{-}$ (siehe Beispiel L) werden in 5 ml absolutem Pyridin gelöst und mehrmals mit Pyridin absolutiert. Dann werden 2,9 µMol $dC^{bz}pTpG^{ibu}pG^{ibu}pA^{bz}pA^{bz}pC^{bz}pTpG^{ibu}_{Bz}$ (siehe Beispiel N) in Pyridin unter nochmaligem Absolutieren zugesetzt, anschließend 0,2 mMol Mesitylensulfonyltetrazolid. Die Reaktion erfolgt 3 Stunden bei Raumtemperatur.

Der Reaktionsabbruch und die Aufarbeitung erfolgt analog Beispiel I.

Ein Viertel des Rohmaterials wird analog Beispiel G detrityliert.

Das Produkt der Detritylierung wird in Pyridin/konz. $NH_3$ 1 : 1 aufgenommen (ca. 8 ml) und 12 Stunden bei 35°C, dann nochmals 6 Stunden bei 55°C stehengelassen. Dann werden alle Lösungsmittel durch Einrotieren im Wasserstrahlvakuum entfernt. Der Rückstand wird in 10 ml Wasser aufgenommen (mit $NH_3$ auf pH 8 eingestellt) und 3 mal mit je 10 ml Ether extrahiert. Die wäßrige Phase wird weitgehend eingeengt und auf eine Biogel P 2 - Säule (60 x 1,7 cm) aufgegeben. Die Elution erfolgt mit Wasser (pH 8).
Ausbeute: ca. 100 $O.D._{260}$.
Charakterisierung:
UV in Wasser: $\lambda_{max}$ = 265 nm; $\lambda_{min}$ = 235 nm.

Die wäßrige Lösung wird auf ca. 50 µl eingeengt und der Reverse-Phase HPLC unterworfen. Säule: µ-Bondapak $C_{18}$ Fa. Waters; Eluans: 0,1 M Triethylammoniumacetatpuffer pH 7 + 12 - 14 % Acetonitril (linearer Gradient innerhalb 7 Minuten); Fluß: 2 ml/Minute. Das Tridecamere wird bei 11 ml Elutionsvolumen eluiert.

Die Produktfraktion wurde eingeengt und über Biogel P 2, wie oben angegeben, entsalzt, dann lyophilisiert.
Ausbeute: 3 O.D.

Charakterisierung:
UV in Wasser: $\lambda_{max}$ = 260 nm; $\lambda_{min}$ = 233 nm.
$R_f$ der DC auf Cellulose
in n-Propanol/$NH_3$/$H_2O$ = 55 : 10 : 35 = 0,15 bez. auf dT
Vergleich:
$R_f$ im gleichen System
des ungeschützten Pentameren = 0,58
Nonameren = 0,28

Die weitere Charakterisierung wurde durch "fingerprint"-Analyse durchgeführt (siehe nachfolgende Abbildung).

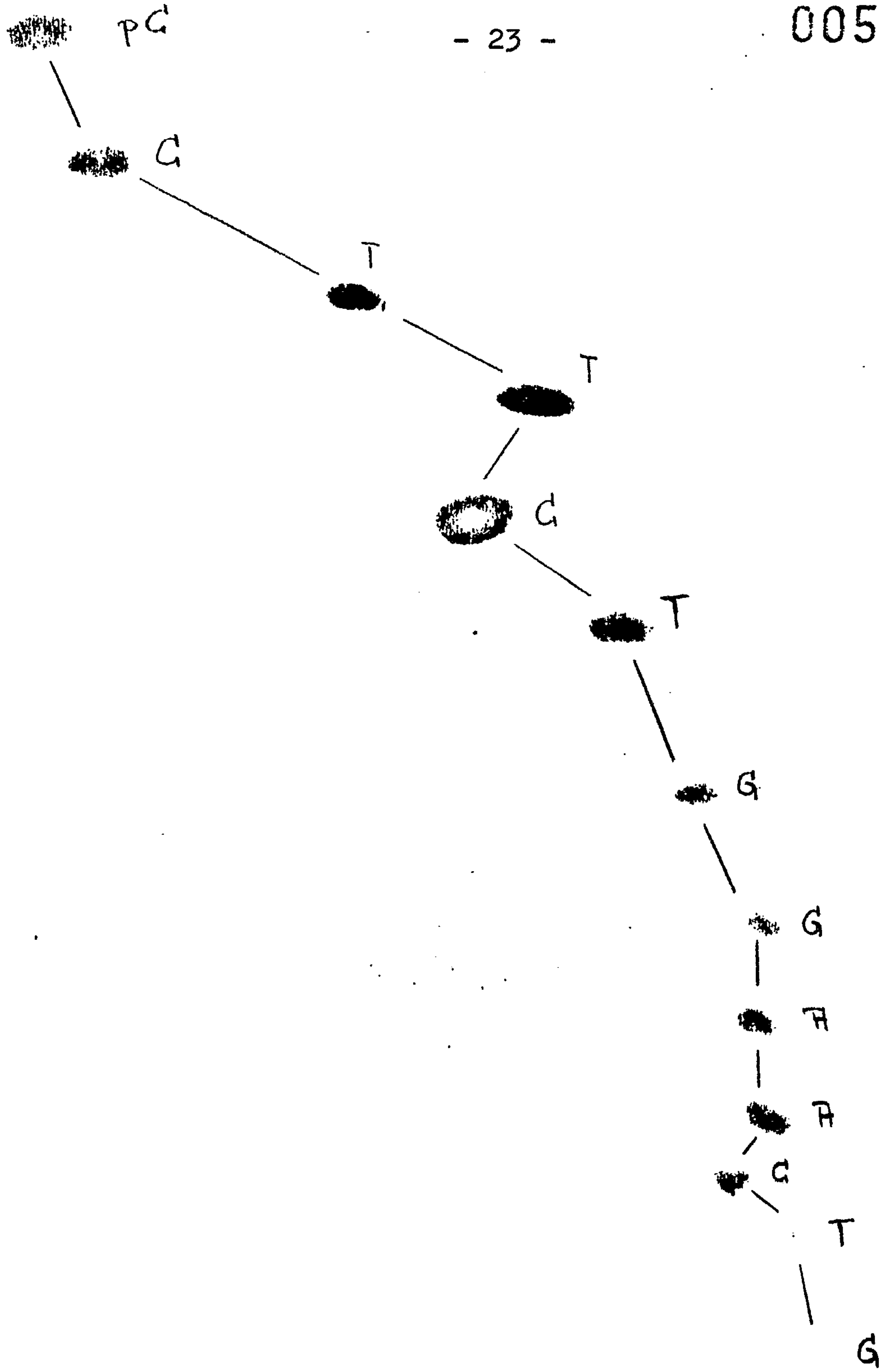
pG
G
T
T
G
T
G
G
A
A
C
T
G

Beispiel 2

dCCTTCTGGAACTG

Hergestellt analog Beispiel 1 durch Umsetzung von 6,3 uMol $DMTrdC^{bz}pC^{bz}pTpTpC^{bz}pTp^{-}$ mit 3,4 uMol $dG^{ibu}pG^{ibu}pA^{bz}pA^{bz}pC^{bz}$-$pTpG^{ibu}_{Bz}$ und 0,2 mMol Mesitylensulfonyltetrazolid.
Charakterisierung der ungeschützten Fragmente.
$R_f$ der DC auf Cellulose
in n-Propanol/$NH_3$/$H_2O$ = 55 : 10 : 35
des ungeschützten Tetrameren dACTG = 0,53
des ungeschützten Heptameren dGGAACTG = 0,25
Charakterisierung:
UV in Wasser: $\lambda_{max}$ = 260 nm; $\lambda_{min}$ = 233 nm.

Das Auffinden interferonspezifischer Sequenzen in Bakterienklonen mit Hilfe des Tridecanukleotids wird gemäß nachfolgendem Beispiel durchgeführt:

I. Herstellung der Hybridisationsprobe

a) 5' Endmarkierung des Tridecanukleotids mit $[\gamma\text{-}^{32}P]$ ATP und T4-Polynukleotid-Kinase.

Die Reaktion erfolgte in einem Volumen von 50 ul, beinhaltend 15 uMol Tridecanucleotid, 50 mM Tris/HCl pH 7.5, 10 mM $MgCl_2$, 5 mM DTT, 0,1 mM Spermidin, 0,1 mM EDTA,

1 mCi $[\gamma\text{-}^{32}P]$ ATP (3000 Ci/mmol) 2 U T4-Polynukleotid-Kinase. Das Reaktionsgemisch wurde 30 Minuten bei 37°C inkubiert, hierauf wurde das endmarkierte Tridecanukleotid vom nicht umgesetzten $^{32}P$-ATP durch Chromatographie auf Sephadex G 25 abgetrennt, mit Alkohol gefällt und in einem kleinen Volumen wieder aufgenommen.

b) cDNA Synthese mit dem endmarkierten Tridecanukleotid als Primer

Die Reaktion erfolgte in einem Volumen von 250 µl, beinhaltend 3 µM endmarkiertes Tridecanuleotid, 0,4 mg/ml poly $(A)^+$ RNA aus mit Sendai-Virus induzierten Namalwazellen, 0,5 mM dNTPs, 50 mM Tris/HCl pH 8,3, 60 mM KCl, 5 mM DTT, 50 µg/ml Actinomycin D, 4 mM $MgCl_2$, 4 mM $Na_4P_2O_7$, 20 U Reverse Transcriptase. Das Reaktionsgemisch wurde 90 Minuten bei 37°C inkubiert. Die synthetisierte cDNA wurde von der Hauptmenge des nicht verlängerten Primers durch Sephadex G 50-Chromatographie abgetrennt, die RNA durch Hydrolyse in 0,3 N Natronlauge, 1 Stunde bei 50°C, entfernt. Die so erhaltene Probe wurde neutralisiert und zur Hybridisierung verwendet.

II. Hybridisierung von Bakterienkolonien mit der endmarkierten, spezifisch geprimten cDNA.

Bakterienklone wurden auf Nitrocellulosefiltern aufgewachsen und nach der Methode von Grunstein und Hogness (Proc. Natl. Acad. Sci. 72, 3961-3965 (1975)) lysiert. Die Filter wurden in 50 % Formamid, 0,6 M NaCl, 0,2 M Tris/HCl pH 8, 20 mM EDTA, 0,02 % Ficoll, 0,02 % Polyvinylpyrrolidon, 0,02 % Bovinserumalbumin, 0,4 mg/ml denaturierter Träger-DNA, 0,1 % $Na_4P_2O_7$, 0,1 % SDS 12 Stunden lang bei 37°C vorhybridisiert, dann wurde die Probe zugegeben und weitere 2 Tage bei 37°C inkubiert. Die oben angegebene Menge an Probe wurde zur Hybridisierung eines Filters der Größe 16 x 25 cm (1600 Klone) verwendet. Nicht hybridisierte Probe wurde durch Waschen in 50 % Formamid, 30 mM NaCl, 3 mM Na-Citrat entfernt. Autoradiographie erfolgte mit Kodak XR-Film und Kodak X-Omatic-Verstärkerfolie.

Ergebnis:

Positive Klone zeigten bereits nach 24 Stunden deutliches Signal.

Damit kann gezeigt werden, daß ein synthetisches Oligonucleotid (13N), welches komplementär zur Basensequenz eines Teilstückes der Interferon RNA (sowohl Leukozyten als auch Fibroblasten-Interferon) ist, zur Identifikation von transformierten Bakterienklonen dienen kann, welche diese Sequenz enthalten.

# Patentansprüche

1. Tridecadeoxynukleotid der Formel

dCCTTCTGGAACTG

2. Verfahren zur Herstellung des Tridecadeoxy-nukleotids gemäß Anspruch 1, dadurch gekennzeichnet, daß geeignete partiell geschützte Oligonukleotidfragmente in Gegenwart eines Kondensationsmittels verknüpft werden und anschließend von dem so erhaltenen voll geschützten Tridecameren die Schutzreste abgespalten werden.

3. Verfahren gemäß Anspruch 2, dadurch gekennzeichnet, daß die Umsetzung in einem wasserfreien Lösungsmittel wie absolutem Pyridin durchgeführt wird.

4. Verfahren gemäß Anspruch 2, dadurch gekennzeichnet, daß als Kondensationsmittel Mesitylensulfonyl-tetrazolid verwendet wird.

5. Verfahren gemäß den Ansprüchen 2, 3 und 4, dadurch gekennzeichnet, daß die Umsetzung bei Temperaturen zwischen 0 und $50^{o}C$, vorzugsweise jedoch bei Raumtemperatur, durchgeführt wird.

6. Verwendung des Tridecadeoxynukleotids gemäß Anspruch 1 als Primer für die Herstellung von komplementären Deoxyribonukleinsäure (cDNA) an Leukozyten-Interferon (IFNα)-mRNA-Matrizen.

7. Verwendung des Tridecadeoxynukleotids gemäß Anspruch 1 als Primer für die Herstellung von komplementären Deoxyribonukleinsäure (cDNA) an Fibroblasten-Interferon (IFNß) mRNA-Matrizen.

8. Verwendung des Tridecadeoxynukleotids gemäß Anspruch 1 als Primer für die Herstellung von komplementären Deoxyribonukleinsäure (cDNA) an Lymphoblasten Interferon (IFNαn)-mRNA-Matrizen.

9. Verwendung des Tridecadeoxynukleotids gemäß Anspruch 1 als Primer für die Herstellung von komplementären Deoxyribonukleinsäure (cDNA) an Immuninterferon (IRNγ)-mRNA-Matrizen.